(19) **Europäisches Patentamt**

**European Patent Office**

**Office européen des brevets**

(11) **EP 1 232 724 B1**

(12) **EUROPEAN PATENT SPECIFICATION**

(45) Date of publication and mention
of the grant of the patent:
**29.03.2006 Bulletin 2006/13**

(51) Int Cl.:
*A61B 5/053* (2006.01)

(21) Application number: **02001911.3**

(22) Date of filing: **30.01.2002**

(54) **Visceral fat measuring apparatus**

Eingeweidefettmessgerät

Dispositif de mesure de la graisse viscérale

(84) Designated Contracting States:
**DE FR GB**

(30) Priority: **15.02.2001 JP 2001037857**

(43) Date of publication of application:
**21.08.2002 Bulletin 2002/34**

(73) Proprietor: **TANITA CORPORATION**
**Tokyo 174 (JP)**

(72) Inventors:
• **Kurata, Soji**
**Itabashi-ku,**
**Tokyo (JP)**

• **Ishikawa, Toshihiko**
**Itabashi-ku,**
**Tokyo (JP)**

(74) Representative: **Müller-Boré & Partner**
**Patentanwälte**
**Grafinger Strasse 2**
**81671 München (DE)**

(56) References cited:
**EP-A- 0 977 150** **EP-A- 1 063 500**

**Description**

BACKGROUND OF THE INVENTION:

Field of the Invention:

The present invention generally relates to a visceral fat measuring apparatus for providing visceral fat information useful for health care of a person.

Prior Art:

In the past, various types of apparatus for providing living body information useful for health care of a person have been proposed and provided. For example, as is disclosed in TOKUKAIHEI No. 10-258043, most of the apparatus for providing the living body information useful for health care are designed to display several types of the living body information and graphically represent evaluation criteria for the living body information.

EP 977 150 A and EP 1 063 500 A describe two devices for estimating the area of visceral fat; the first one uses tomographic images, the second one uses measurements of body impedance and A-mode ultrasound images.

However, because of the prior art apparatus for providing the living body information useful for health care designed to display several types of the living body information, as described above, it is difficult for a user to correctly identify what living body information is important for the health. In addition, it is also difficult for the user to promptly understand which criterion corresponds to which living body information. Therefore, there is a need to solve those problems in the prior art, especially for the apparatus for providing such visceral fat information that has not yet been in popular, but is highly correlated to the health condition.

In view of the above, an object of the present invention is to provide a visceral fat measuring apparatus for providing the information that is readily understandable and identifiable by a user.

SUMMARY OF THE INVENTION:

In order to achieve above object, the present invention provides a visceral fat measuring apparatus for providing living body information including visceral fat information, comprising a display unit, whereby said display unit displays the visceral fat information in such manner that visceral fat area is reduced by a fixed rate to produce a reduced level value for the visceral fat area which is then displayed as the visceral fat information. In this way, displaying such reduced level value for the visceral fat area allows reduction in number of digits for representing the visceral fat area. Therefore, a user can easily identify and understand such reduced level value.

According to one embodiment of the present invention said display unit displays the reduced level value for the visceral fat area, while switching with the display of other living body information than the visceral fat information. In such manner, by displaying the reduced level value, while switching with the display of other living body information than the visceral fat information, the level value can be displayed independent of other living body information than the visceral fat information so that the user can see each of them separately. In other words, the user can see only visceral fat area (or the level value) that is highly correlated to the health condition, and therefore, user can promptly understand it.

According to another aspect of the present invention, there is provided a visceral fat measuring apparatus for providing living body information including visceral fat information, comprising a display unit, whereby said display unit displays the visceral fat information and has a display window frame formed thereon, and said display unit displays evaluation result for visceral fat area in the form of bar graph whose length is increased/decreased in stepwise from one side to another side of said display window frame. In this way, by displaying the evaluation result for the visceral fat area with the bar graph whose length is increased/decreased in stepwise from one side to another side of the display window frame, the display window frame itself acts as the basis for evaluation of the visceral fat area. In addition, the display window frame has a dear boundary between inside and outside of the display window. Therefore, the user can easily see the bar graph and readily understand the evaluation result with higher impression.

According to one embodiment of the present invention said bar graph consists of a single bar whose length is increased/decreased in stepwise from one side to another side of the display window frame along further side present therebetween. Therefore, the user can see and confirm the increasing/decreasing of the bar graph along said further side present between said one side and said another side of the display window frame. This is significantly useful to assist the user's understanding.

According to another aspect of the present invention, there is provided a visceral fat measuring apparatus for providing living body information including visceral fat information, comprising a display unit, whereby said display unit displays the visceral fat information in such manner that the current visceral fat information and the previous visceral fat information are concurrently displayed. In this way, by concurrently displaying the current visceral fat information and the previous visceral fat information, the user can compare both current and previous information as to their transition.

According to one embodiment of the present invention said display unit displays the current visceral fat information and the previous visceral fat information in the different size. In this way, displaying the current and the previous information in different size allows for the user to clearly distinguish therebetween.

According to further aspect of the present invention, there is provided a visceral fat measuring apparatus for providing living body information including visceral fat information, comprising a display unit, whereby said display unit displays the visceral fat information in such manner that the evaluation result for the visceral fat area is indicated generally in the form of an oval. The term "generally in the form of an oval" as used herein means any one of the shapes such as an oval, a part of an oval, and an oval with a portion modified. In this way, displaying the evaluation result for the visceral fat area generally in the form of an oval causes the user to imagine a slice of the abdomen wherein the visceral fat area is positioned, and emphasizes that it is the evaluation result for the visceral fat area. This is very effective for the user to promptly understand the evaluation result.

According to one embodiment of the present invention said display unit changes the inside condition of the oval in stepwise depending on the visceral fat area. In this way, changing the inside condition of the oval in stepwise emphasizes that it is the evaluation result for the visceral fat area. This is very effective for the user to promptly understand the evaluation result.

BRIEF DESCRIPTION OF THE DRAWINGS:

Now, the present invention will be described in more detail with reference to the accompanying drawings, in which:

Fig. 1 is an external view illustrating a visceral fat measuring apparatus according to one embodiment of the present invention;
Fig. 2 is a block diagram illustrating a configuration of the visceral fat measuring apparatus;
Fig. 3 is a flow chart illustrating sequential steps of operation of the visceral fat measuring apparatus;
Figs. 4(a), 4(b) and 4(c) illustrate an exemplary display of level value representing visceral fat information;
Figs. 5(a), 5(b) and 5(c) illustrate another exemplary display of level value representing visceral fat information; and
Figs. 6(a), 6(b) and 6(c) illustrate further exemplary display of level value representing visceral fat information.

DESCRIPTION OF THE PREFERRED EMBODIMENTS:

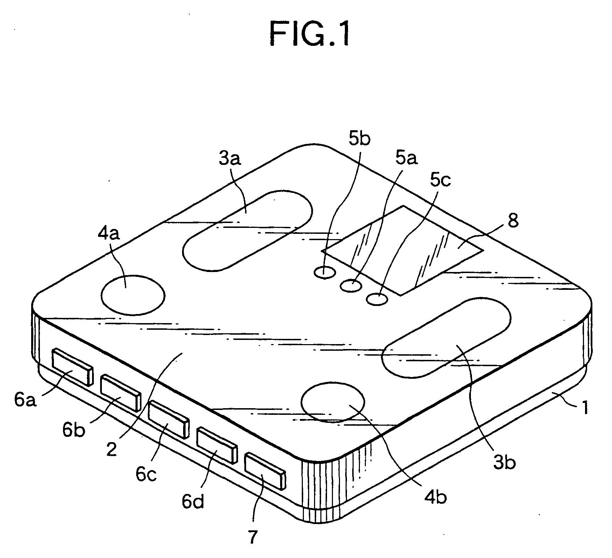

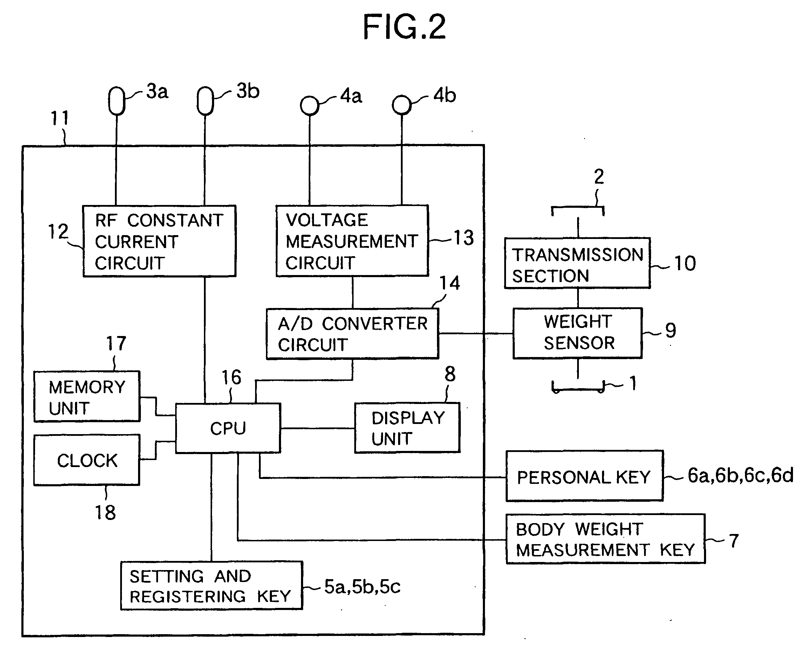

Fig. 1 is an external view illustrating a visceral fat measuring apparatus according to one embodiment of the present invention. Fig. 2 is a block diagram illustrating a configuration of the visceral fat measuring apparatus. As can be seen in Fig. 1, the visceral fat measuring apparatus comprises: a base 1 that is a foundation of the apparatus; a platform 2 on which a living body mounts; current feeding electrodes 3a, 3b for forming an electric current path in the living body; measurement electrodes 4a, 4b for detecting potential difference produced in the living body wherein the current path is formed between the current feeding electrodes 3a, 3b; setting and registration keys 5a, 5b, 5c for setting and registering the living body specific information and the time; personal keys 6a, 6b, 6c, 6d for retrieving the living body specific information entered by the setting and registration keys 5a, 5b, 5c for measurement; a body weight measurement key 7 for measuring the body weight; and a display unit 8 for displaying various information entered by the setting and registration keys 5a, 5b, 5c as well as the measurement results of body weight, body fat and visceral fat.

The visceral fat measuring apparatus further comprises: within a compartment defined by the base 1 and the platform 2, a weight sensor 9 for detecting a load and for producing an electric signal in response thereto; a transmission section 10 for transmitting the load applied to the platform 2 to the weight sensor 9; and an electronic circuit board 11.

The electronic circuit board 11 comprises: the display unit 8 and the setting and registration keys 5a, 5b, 5c mounted on the platform 2; an RF constant current circuit 12 for supplying small RF constant current to the current feeding electrodes 3a, 3b; a voltage measurement circuit 13 for measuring potential difference of the living body produced between the measurement electrodes 4a, 4b; an A/D converter circuit 14 for converting an analogue signal from the voltage measurement circuit 13 and the weight sensor 9 into a digital signal; a clock 15 for producing a timing signal; a CPU 16 for performing an arithmetic operation based on various living body information such as body weight, body fat and visceral fat and for making an evaluation for the arithmetic result and controlling the operation of entire apparatus; and a memory unit 17 for storing the measurement data for use in estimation of visceral fat area, such as body weight and body fat rate as calculated by the CPU 16, the living body specific data entered by the setting and registration keys 5a, 5b, 5c, as well as a regression formula created on the basis of such data in advance for estimating several types of visceral fat areas.

The current feeding electrodes 3a, 3b, the measurement electrodes 4a, 4b, the weight sensor 9, the personal keys 6a, 6b, 6c, 6d, the body weight measurement key 7, and the electronic circuit board 11 are connected with the wire lines, as shown in Fig. 2, to configure the entire apparatus.

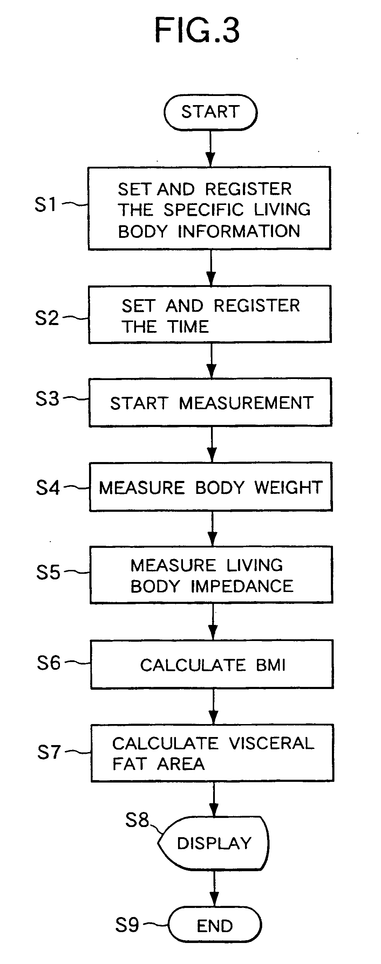

Now, an operation of the visceral fat measuring apparatus according to the present invention will be described. Fig. 3 is a flow chart illustrating sequential steps of operation of the visceral fat measuring apparatus. First of all, in step S1, a user or a person to be measured sets and registers his or her specific body information such as age, sex, height, physical feature and waist size when using the visceral fat measuring apparatus for the first time or when updating the previously registered data. The procedure for setting and registering such information proceeds as follows: First, the setting and registering key 5a is depressed to proceed to a living body information entering mode wherein certain number is selected with the setting and registering keys 5b and 5c for registering the specific living body information for the user. Then the setting and registering key 5a is again depressed to determine that the data item to be entered has been selected. Thereafter, certain numerical value or the relevant item is selected with the setting and registering keys 5b and 5c, and then, the setting and registering key 5a is again depressed to determine that the data has been entered. The determined data and the item are stored in the memory unit 17.

Then, in step S2, the user sets and registers the time when using the visceral fat measuring apparatus for the first time or when updating the previously registered data. The procedure for setting and registering the time proceeds as follows: the setting and registering key 5a and any one of the setting and registering keys 5b or 5c are depressed at the same time to proceed to a date entering mode wherein certain numerical value is selected with the setting and registering key 5b or 5c according to the guidance of the cursor on the display unit, and then, the setting and registering key 5a is depressed to determine that the relevant data has been entered.

Next, in step S3, any one of the personal keys 6a, 6b, 6c and 6d corresponding to the number that has been selected in setting and registering the specific living body information in step S1 is depressed. Then the number "0" is displayed on the display unit 8 to proceed to a measuring mode of operation.

Next, in step S4, the user mounts on the platform 2 for body weight measurement with his left foot contact to the current feeding electrode 3a and the measurement electrode 4a and his right foot contact to the current feeding electrode 3b and the measurement electrode 4b. Then the body weight is derived according to the prior art weighting technique, stored in the memory 17 and displayed on the display unit 8.

Next, in step S5, the user continues to mount on the platform 2 for living body impedance measurement. Then the body fat mass, the fat free mass and the body fat rate are derived based on the measured living body impedance according to the prior art body fat measuring technique, and stored in the memory unit 17. The body fat rate is displayed on the display unit 8.

Next, in step S6, the CPU 16 calculates "BMI" based on the height that has been set and registered as the specific living body information in step S1 and on the body weight that has been measured in step S4.

Thereafter, in step S7, the CPU 16 selects, among the regression formulae or equations (1) to (4) as described below and stored in the memory unit 17 for estimating the visceral fat area, the equations (1) to (3) if the specific living body information that has been set and registered in step S1 is for a man, or the equation (4) if it is for a woman.

$$\text{Visceral Fat Area } VAF_1 = X_1 \times \text{waist} + X_2 \times (\text{height}^2 / \text{fat free mass})$$
$$+ X_3 \times (\text{body fat mass} / \text{height}^2)^2 + X_4 \times BMI + Y_1 \qquad (1)$$

$$\text{Visceral Fat Area } VAF_2 = X_5 \times VAF_1 + X_6 \times \text{age} + Y_2 \qquad (2)$$

$$\text{Visceral Fat Area } VAF_3 = X_7 \times VAF_2 + X_8 \times (100 - \text{body fat rate}) + Y_3 \qquad (3)$$

$$\text{Visceral Fat Area } VAF_4 = X_9 \times \text{waist} + X_{10} \times (1 / \text{body fat mass})$$
$$+ X_{11} \times BMI + X_{12} \times \text{age} + Y_4 \qquad (4)$$

where $X_1$ to $X_{12}$ and $Y_1$ to $Y_4$ are constant that have been predetermined to have correlation to each other.

Thereafter, the relevant parameters are selected from among the specific living body information such as height, waist size and age that have been set and registered in step S1; the body weight that has been measured in step S4;

the body fat mass, fat free mass and body fat rate that have been derived in step S5; and the BMI that has been derived in step S6, and then, they are substituted for the corresponding items of the regression equations preselected for a man or a woman in order to derive the visceral fat area (in the unit of $cm^2$). In particular, for the visceral fat area for a man, the visceral fat area $VFA_1$ is calculated using the equation (1), then the visceral fat area $VFA_2$ is calculated using the equation (2) that includes the visceral fat area $VFA_1$ as the parameter, and finally the visceral fat area $VFA_3$ is calculated using the equation (3) that includes the visceral fat area $VFA_2$, as the parameter.

Next, the visceral fat area thus calculated is multiplied by a fixed rate of 0.1 to produce a reduced level value for the visceral fat area, which level value is then output to the display unit 8 together with the result of evaluation for the visceral fat area.

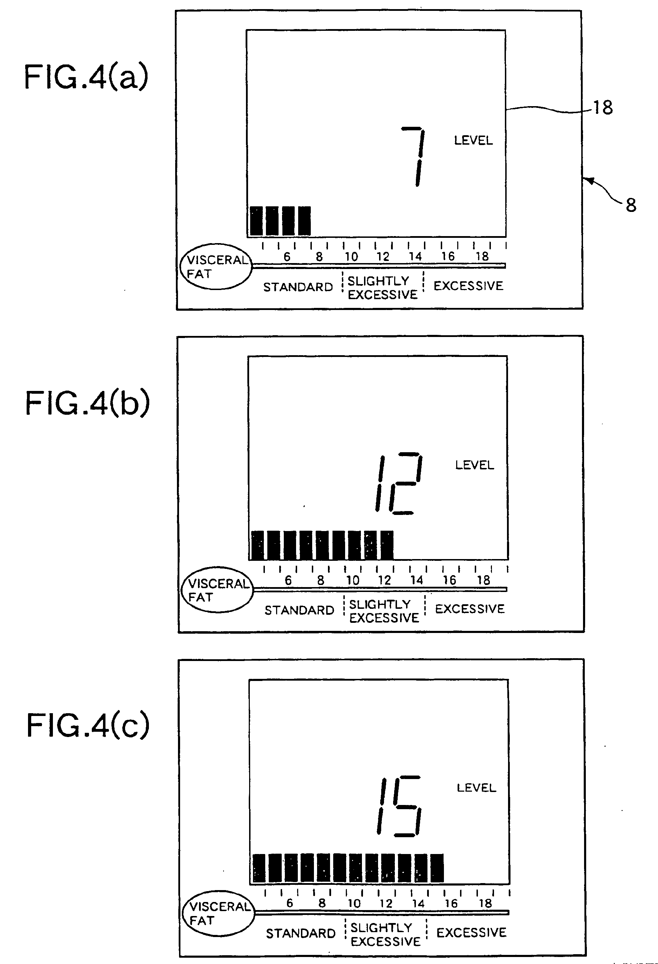

Thereafter, in step S8, the reduced level value for the visceral fat area and the result of evaluation are displayed on the display unit 8 that is the essential element of the present invention. Then, an operation of the display unit 8 will be described in detail with reference to Figs. 4 to 6.

Figs. 4 (a), 4(b) and 4(c) illustrate an exemplary display produced on the display unit 8 which indicates a level value for the visceral fat information, while switching with another display of other living body information than the visceral fat information, such as body weight and body fat rate. In this example the display unit 8 includes a display window frame 18. Outside the display window frame 18, a scale for showing the level value in the form of bar graph and a three-step evaluation result indication area are printed beneath the bottom side of the display window frame 18. The evaluation result is indicated by three steps consisting of "Normal" which means that the level value is not greater than 9; "Slightly Excessive" which means that the level value is in the range of 10 to 14; and "Excessive" which means that the level value is not less than 15. Inside the display window frame 18, the reduced level value for the visceral fat area that is reduced by multiplication of the fixed rate of 0.1 is displayed, while switching with other display of body weight and body fat rate measured in steps S4 and S5. In addition, the bar graph showing the reduced level value runs from the left-hand side toward the right-hand side of the display window frame 18.

Displaying such reduced level value for the visceral fat area on the display unit 8 allows reduction in number of digits for representing the visceral fat area. In addition, by displaying such reduced level value for the visceral fat area, while switching with another display of the body weight and body fat rate, the level value can be displayed independent of the body weight and body fat rate so that the user can clearly see only the level value for the visceral fat area. Furthermore, by displaying the evaluation result for the visceral fat area with the bar graph whose length is increased/ decreased in stepwise from one side to another side of the display window frame 18, the user can easily see and promptly understand the evaluation result with higher impression, because the display window frame 18 itself acts as the basis for evaluation of the visceral fat area and the display window frame 18 has a clear boundary between inside and outside of the display window. In particular the bar graph consists of a single bar whose length is increased/decreased in stepwise from one side to another side of the display window frame 18 along further side present therebetween. Therefore, the user can see and confirm the increasing/decreasing of the bar graph along said further side present between said one side and said another side of the display window frame. This is very effective for the user to promptly understand the evaluation result.

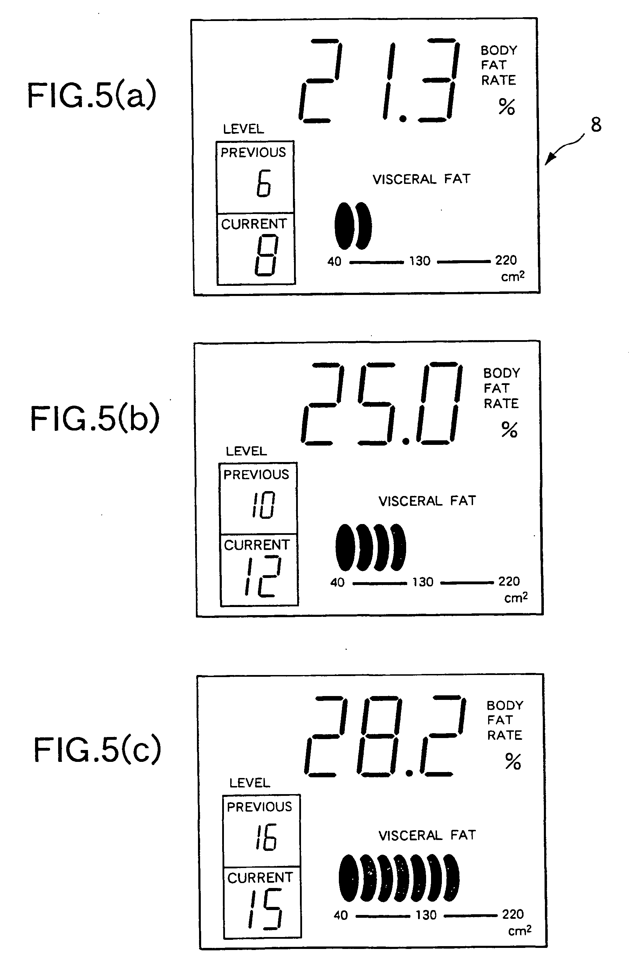

Figs. 5 (a), 5(b) and 5(c) illustrate another exemplary display produced on the display unit 8 which concurrently indicates the previous visceral fat information and the current visceral fat information in different character size. In this example, the display unit 8 alternately displays the body weight and the body fat rate, and at the same time, displays the current level value for the visceral fat area in character size greater than that of the previous level value. In addition, the evaluation result for the current level value is displayed in bar graph whose length is increased/decreased in stepwise. A segment for indicating the increment/decrement of the bar graph is displayed generally in the form of an oval, and a scale between a start point and an end point of the bar graph indicates the visceral fat area in the unit of $cm^2$. The term "generally in the form of an oval" as used herein means any one of the shapes such as an oval, a p art of an oval, and an oval with a portion modified.

Displaying the current and the previous level values in the different character size on the display 8 allows for the user to dearly distinguish therebetween and to compare them for transition. Furthermore, displaying the evaluation result for the current level value in the form of an oval causes the user to imagine a slice of the abdomen wherein the visceral fat area is positioned, and emphasizes that it is the evaluation result for the visceral fat area. This is very effective for the user to promptly understand the evaluation result.

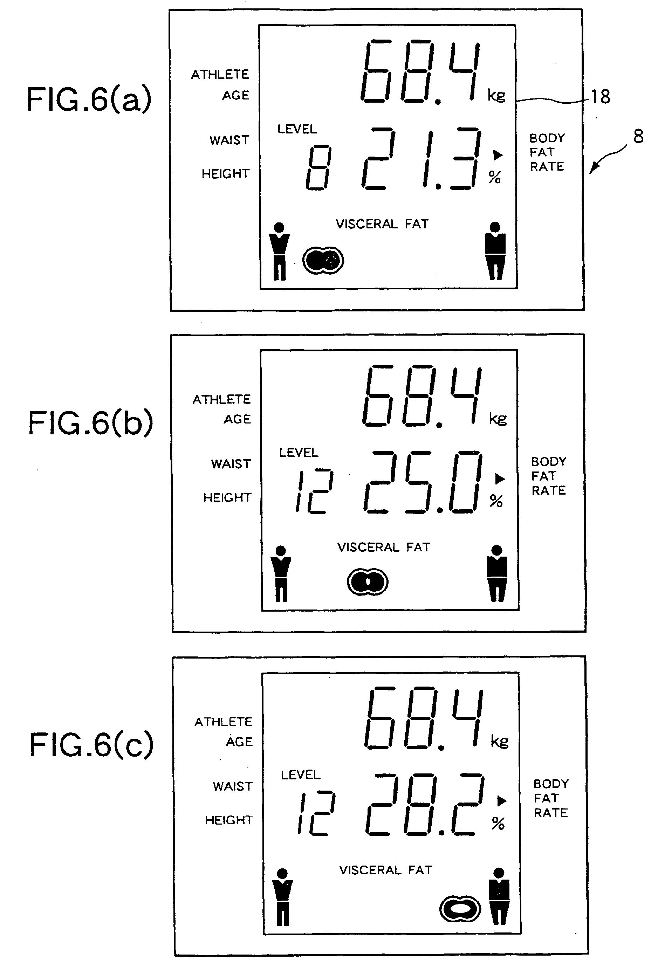

Figs. 6 (a), 6(b) and 6(c) illustrate a further exemplary display produced on the display unit 8 which indicates the evaluation result for the visceral fat area in such manner that a slice mark generally in the form of an oval is changed in its inside condition in stepwise depending on the visceral fat area. In this example the display unit 8 has a display window frame 18. Outside the display window frame, certain items corresponding to the content of the display are printed. On the other hand, inside the display window frame, the body weight is displayed on the top portion, the level value for the visceral fat area is displayed on the left-hand side of the middle portion, and the body fat rate derived from the specific living body information that has been entered as the numerical value and the living body impedance that has been measured is displayed on the right-hand side of the middle portion. In this connection, a triangle mark positioned at right most end of the middle portion and adjacent the item printed outside the display window frame is turned ON or flashed. Furthermore, the evaluation result for the level value is displayed on the bottom portion with the slice mark generally in the form of an oval. The slice mark is changed in its inside condition (in particular the size of blank or white portion) in stepwise depending on the level value for the visceral fat area derived.

Displaying the evaluation result for the visceral fat area generally in the form of an oval on the display unit 8, while changing the inside condition of the oval in stepwise, causes the user to imagine a slice of the abdomen wherein the visceral fat area is positioned, and emphasizes that it is the evaluation result for the visceral fat area. This is very effective for the user to promptly understand the evaluation result.

Finally, in step S9 of Fig. 3, the user confirms the display on the screen and performs the procedure for ending the operation of the apparatus.

In the above description the visceral fat information has been described to mean the visceral fat area. However, the visceral fat information may include other visceral fat values such as visceral fat mass, visceral fat rate, etc., within the scope of the present invention.

It is apparent from the foregoing that the visceral fat information that has not yet been in popular, but is highly correlated to the health condition can easily be distinguished from other living body information and can easily be understood by a user, because the display of such visceral fat information can be seen and identified by the person without any difficulty.

**Claims**

1. A visceral fat measuring apparatus for providing living body information including visceral fat information, comprising a display unit for displaying a visceral fat area as a visceral fat information in the form of a reduced level value corresponding to the visceral fat area as expressed in square centimetres and reduced by a fixed rate.

2. A visceral fat measuring apparatus according to claim 1 in which said display unit is suitable for displaying the reduced level value for the visceral fat area, while switching with the display of other living body information than the visceral fat information.

3. A visceral fat measuring apparatus according to claim 1 in which said display unit has a display window frame formed thereon, said display unit being suitable for displaying the reduced level value in the form of bar graph whose length increasing/decreasing in stepwise from one side to the other side of said display window frame.

4. A visceral fat measuring apparatus according to claim 1 in which said display unit is suitable for displaying concurrently the current level value and the previous level value.

5. A visceral fat measuring apparatus according to claim 4 in which said display unit is suitable for displaying the current level value and the previous level value in the different size.

6. A visceral fat measuring apparatus according to claim 1 in which said display unit is suitable for displaying the level value in a graph generally in the form of an oval.

7. A visceral fat measuring apparatus according to claim 6 in which said graph changes the inside condition of the oval in stepwise depending on the visceral fat area.

8. A visceral fat measuring apparatus according to claim 3 in which said bar graph consists of a single bar whose length is increased/decreased in stepwise from one side to another side of the display window frame along further side present therebetween.

9. A visceral fat measuring apparatus according to claim 1 in which said level value is a numerical one.

10. A visceral fat measuring apparatus according to claim 2 in which, when switched from the display of other living body information than the visceral fat information, said other living body information than the visceral fat information is not displayed.

11. A visceral fat measuring apparatus according to claim 8 in which said further side has a scale.

**Patentansprüche**

1. Eingeweide- bzw. Viszeralfettmeßgerät zum Bereitstellen von Information über einen lebenden Körper, beinhaltend Eingeweidefettinformation bzw. Information über viszerales Fett, umfassend eine Anzeigeeinheit zum Anzeigen eines Eingeweidefettbereichs als eine Eingeweidefettinformation in der Form eines Werts eines reduzierten Niveaus entsprechend dem Eingeweidefettbereich, ausgedrückt in Quadratzentimeter, und um eine feststehende Rate bzw. Größe reduziert.

2. Eingeweidefettmeßgerät nach Anspruch 1, in welchem die Anzeigeeinheit zum Anzeigen des Werts des reduzierten Niveaus für den Eingeweidefettbereich geeignet ist, während mit der Anzeige von anderer Information betreffend den lebenden Körper als die Eingeweidefettinformation umgeschaltet ist bzw. wird.

3. Eingeweidefettmeßgerät nach Anspruch 1, in welchem die Anzeigeeinheit einen Anzeigefensterrahmen darauf ausgebildet aufweist, wobei die Anzeigeeinheit für ein Anzeigen des Werts reduzierten Niveaus in der Form eines Balkendiagramms geeignet ist, dessen Länge stufenweise von einer Seite zu der anderen Seite des Anzeigefensterrahmens ansteigt/absinkt.

4. Eingeweidefertmeßgerät nach Anspruch 1, in welchem die Anzeigeeinheit zum gleichzeitigen Anzeigen des Werts des gegenwärtigen Niveaus und des Werts des vorherigen Niveaus geeignet ist.

5. Eingeweidefettmeßgerät nach Anspruch 4, in welchem die Anzeigeeinheit zum Anzeigen des Werts des gegenwärtigen Niveaus und des Werts des vorherigen Niveaus in der unterschiedlichen Größe geeignet ist.

6. Eingeweidefettmeßgerät nach Anspruch 1, in welchem die Anzeigeeinheit zum Anzeigen des Niveauwerts in einem Graph bzw. Diagramm allgemein in der Form eines Ovals geeignet ist.

7. Eingeweidefettmeßgerät nach Anspruch 6, in welchem der Graph die innere Bedingung des Ovals stufenweise in Abhängigkeit von dem Eingeweidefettbereich verändert.

8. Eingeweidefettmeßgerät nach Anspruch 3, in welchem das Balkendiagramm aus einem einzigen Balken besteht, dessen Länge stufenweise von einer Seite zur anderen Seite des Anzeigefensterrahmens entlang einer weiteren Seite, die dazwischen vorhanden ist, ansteigt/absinkt.

9. Eingeweidefettmeßgerät nach Anspruch 1, in welchem der Niveauwert ein numerischer ist.

10. Eingeweidefettmeßgerät nach Anspruch 2, in welchem, wenn von der Anzeige von anderer information, betreffend den lebenden Körper statt der Eingeweidefettinformation umgeschaltet ist bzw. wird, die andere Information betreffend den lebenden Körper als die Körperfettinformation nicht angezeigt ist.

11. Eingeweidefettmeßgerät nach Anspruch 8, in welchem die weitere Seite eine Skala aufweist.


**Revendications**

1. Dispositif de mesure de la graisse viscérale pour fournir des informations corporelles vitales comprenant des informations sur la graisse viscérale, comprenant une unité pour afficher une surface de graisse viscérale comme les informations sur la graisse viscérale sous la forme d'une valeur de niveau réduite correspondant à la surface de graisse viscérale exprimée en centimètres carrés et réduite d'un taux fixe.

2. Dispositif de mesure de la graisse viscérale selon la revendication 1, dans lequel ladite unité d'affichage est adaptée pour afficher la valeur de niveau réduite pour la surface de graisse viscérale, tout en commutant avec l'affichage d'autres informations corporelles vitales que les informations sur la graisse viscérale.

3. Dispositif de mesure de la graisse viscérale selon la revendication 1, dans lequel ladite unité d'affichage a un cadre de fenêtre d'affichage formé sur celle-ci, ladite unité d'affichage étant adaptée pour afficher la valeur de niveau réduite sous la forme de graphique à barres dont la longueur augmente/diminue par échelons d'un côté à l'autre côté dudit cadre de fenêtre d'affichage.

4. Dispositif de mesure de la graisse viscérale selon la revendication 1, dans lequel ladite unité d'affichage est adaptée pour afficher simultanément la valeur de niveau actuelle et la valeur de niveau précédente.

5. Dispositif de mesure de la graisse viscérale selon la revendication 4, dans lequel ladite unité d'affichage est adaptée pour afficher la valeur de niveau actuelle et la valeur de niveau précédente avec une taille différente.

6. Dispositif de mesure de la graisse viscérale selon la revendication 1, dans lequel ladite unité d'affichage est adaptée pour afficher la valeur de niveau dans un graphique généralement sous la forme d'un ovale.

7. Dispositif de mesure de la graisse viscérale selon la revendication 6, dans lequel ledit graphique change la condition interne de l'ovale par échelons en fonction de la surface de graisse viscérale.

8. Dispositif de mesure de la graisse viscérale selon la revendication 3, dans lequel ledit graphique à barres est constitué d'une unique barre dont la longueur est augmentée/diminuée par échelons d'un côté à un autre côté du cadre de fenêtre d'affichage suivant un côté ultérieur présent entre ceux-ci.

9. Dispositif de mesure de la graisse viscérale selon la revendication 1, dans lequel ladite valeur de niveau est une valeur numérique.

10. Dispositif de mesure de la graisse viscérale selon la revendication 2, dans lequel, quand il est commuté à partir de l'affichage d'informations corporelles vitales autres que les informations sur la graisse viscérale, lesdites informations corporelles viscérales autres que les informations sur la graisse viscérale ne sont pas affichées.

11. Dispositif de mesure de la graisse viscérale selon la revendication 8, dans lequel ledit côté ultérieur a une échelle.

# FIG.1

# FIG.2

# FIG.3

```
                    ┌─────────┐
                    │  START  │
                    └─────────┘
                         │
                         ▼
            ┌──────────────────────────┐
            │  SET AND REGISTER        │
     S1     │  THE SPECIFIC LIVING     │
            │  BODY INFORMATION        │
            └──────────────────────────┘
                         │
                         ▼
            ┌──────────────────────────┐
     S2     │  SET AND REGISTER        │
            │  THE TIME                │
            └──────────────────────────┘
                         │
                         ▼
            ┌──────────────────────────┐
     S3     │  START MEASUREMENT       │
            └──────────────────────────┘
                         │
                         ▼
            ┌──────────────────────────┐
     S4     │  MEASURE BODY WEIGHT     │
            └──────────────────────────┘
                         │
                         ▼
            ┌──────────────────────────┐
     S5     │  MEASURE LIVING          │
            │  BODY IMPEDANCE          │
            └──────────────────────────┘
                         │
                         ▼
            ┌──────────────────────────┐
     S6     │  CALCULATE BMI           │
            └──────────────────────────┘
                         │
                         ▼
            ┌──────────────────────────┐
     S7     │  CALCULATE VISCERAL      │
            │  FAT AREA                │
            └──────────────────────────┘
                         │
                         ▼
     S8        ┌──────────────┐
               │   DISPLAY    │
               └──────────────┘
                         │
                         ▼
     S9           ┌─────────┐
                  │   END   │
                  └─────────┘
```

12

**FIG.5(a)**

BODY FAT RATE %

21.3

8

LEVEL

PREVIOUS
6

CURRENT
8

VISCERAL FAT

40 ——— 130 ——— 220
cm²

**FIG.5(b)**

BODY FAT RATE %

25.0

LEVEL

PREVIOUS
10

CURRENT
12

VISCERAL FAT

40 ——— 130 ——— 220
cm²

**FIG.5(c)**

BODY FAT RATE %

28.2

LEVEL

PREVIOUS
16

CURRENT
15

VISCERAL FAT

40 ——— 130 ——— 220
cm²

FIG.6(a)

ATHLETE
AGE

WAIST

HEIGHT

LEVEL

*68.4* kg

*8* *21.3* ▶ %

VISCERAL FAT

BODY
FAT
RATE

18

8

FIG.6(b)

ATHLETE
AGE

WAIST

HEIGHT

LEVEL

*68.4* kg

*12* *25.0* ▶ %

VISCERAL FAT

BODY
FAT
RATE

FIG.6(c)

ATHLETE
AGE

WAIST

HEIGHT

LEVEL

*68.4* kg

*12* *28.2* ▶ %

VISCERAL FAT

BODY
FAT
RATE